# EUROPEAN PATENT SPECIFICATION

(11) **EP 4 117 622 B1**
(45) Date of publication and mention of the grant of the patent: **20.05.2026**
(21) Application number: 21710328.2
(22) Date of filing: 12.03.2021
(51) Int. Cl.: A61K 9/00, A61P 27/04, A61K 9/06, A61K 47/10

(54) **NEW USE AND METHOD OF TREATMENT**
NEUE VERWENDUNG UND VERFAHREN ZUR BEHANDLUNG
NOUVELLE UTILISATION ET NOUVEAU PROCÉDÉ DE TRAITEMENT

(30) Priority: 12.03.2020 EP 20162821
(43) Date of publication of application: 18.01.2023
(73) Proprietor: Hunter Capital AB (publ), 114 51 Stockholm (SE)
(72) Inventor: VIDAEUS, Martin, 118 60 Stockholm (SE); AGELAND, Hans, 118 60 Stockholm (SE); BJÖRKLUND, Ulf, 118 60 Stockholm (SE); GARHÖFER, Gerhard, 118 60 Stockholm (SE)
(74) Representative: AWA Sweden AB
(86) International application number: PCT/EP2021/056314
(87) International publication number: WO 2021/180913

(56) References cited:
- EP-A1- 0 551 626
- CN-A- 108 295 318
- US-A1- 2017 112 936
- US-A1- 2018 098 937
- ANUMOLU S S ET AL: "Doxycycline loaded poly(ethylene glycol) hydrogels for healing vesicant-induced ocular wounds", BIOMATERIALS, ELSEVIER, AMSTERDAM, NL, vol. 31, no. 5, 1 February 2010 (2010-02-01), pages 964 - 974, XP026790426, ISSN: 0142-9612, [retrieved on 20091022]
- SOLIMAN KARIM A ET AL: "Poloxamer-based in situ gelling thermoresponsive systems for ocular drug delivery applications", DRUG DISCOVERY TODAY, ELSEVIER, AMSTERDAM, NL, vol. 24, no. 8, 5 June 2019 (2019-06-05), pages 1575 - 1586, XP085782057, ISSN: 1359-6446, [retrieved on 20190605], DOI: 10.1016/J.DRUDIS.2019.05.036

## Description

### Field of the invention

The present invention is in the field of pharmaceutical compositions for administration in the eye of mammalian subjects. In particular, it relates to a pharmaceutical composition for use in, and a method for, the ophthalmic treatment of dry eye disease.

### Background

According to the non-profit organization Tear Film & Ocular Surface Society, dry eye disease (DED), interchangeably denoted "dry eye syndrome", is a multifactorial disease of the ocular surface characterized by a loss of homeostasis of the tear film, and accompanied by ocular symptoms, in which tear film instability and hyperosmolarity, ocular surface inflammation and damage, and neurosensory abnormalities play etiological roles (Craig et al (2017), The Ocular Surface 15:802-812).

DED is widespread in the human population worldwide, but rates of prevalence differ between countries mainly as a result of differing definitions and patient inclusion criteria (Stapleton et al (2017), The Ocular Surface 15:334-365).

Available treatments for dry eye disease are reviewed in Gomes and Santo (2019), The Ocular Surface 17:9-19 (see Table 1).

Slow-release formulations based on a reversibly thermo-reversible gel of a block copolymer and an associative gelling agent are disclosed in e.g. US9592295. While many different applications and uses for the disclosed hydrogels are given in US9592295, all of these applications and uses rely on the use of the hydrogel as carrier or vehicle for delivery and release of active ingredients, which are disclosed as external to the actual hydrogel. There is no disclosure in US9592295 of any use of the gel itself as active ingredient, and no indication that it may have a therapeutic effect in its own right.

A phase II clinical trial identified as NCT03821415 investigated the use of a hydrogel as disclosed in US9592295 as a drug delivery vehicle or carrier for the active ingredient 17β-estradiol-3-phosphate in the treatment of dry eye disease. In the setup of this clinical trial, the hydrogel without active ingredient was used as placebo, i.e. a use based on the understanding that the hydrogel formulation *per se* does not have any therapeutic effect. The results of the clinical trial in question have not been reported. US 2018/098937 discloses artificial tear compositions used e.g. to treat e.g. dry eye, wet age-related macular degenerations or diabetes, comprises at least one nonionic surfactant, and at least one excipient comprising viscosity enhancer, polyol or electrolyte.

There is a need in the art for a convenient and effective formulation for treatment of dry eye disease in mammalian subjects in need thereof, and for novel methods to treat such a condition.

### Disclosure of the invention

It is an object of the present invention to meet this need. Other objects are apparent to the skilled person from the disclosure.

Thus, in a first aspect, the disclosure provides a pharmaceutical composition, which comprises a reversely thermo-reversible hydrogel as active ingredient, said hydrogel comprising, by weight percent of the total composition:
- about 5-25 % of a water soluble block copolymer comprising at least two blocks of polyethylene oxide and at least one block of polypropylene oxide; and
- about 0.05-10 % of at least one associative gelling agent having a water solubility of less than 0.5 g / 100 ml at 20 °C and being capable of forming water soluble inter-molecular complexes with said water soluble block copolymers in water;

wherein said composition does not comprise any additional active ingredient,
for use in the treatment of dry eye disease by local administration in the eye of a mammalian subject in need thereof.

By the use of a reversely thermo-reversible hydrogel as defined herein, it is possible to provide a pharmaceutical composition which is liquid at normal room temperature, but which forms a gel at the higher temperatures of a mammalian body, e.g. from 32 °C and upwards. The beneficial gelling properties of the pharmaceutical composition make for ease of application in the desired ocular environment of the subject. In particular, these properties allow for convenient storage, handling and administration of the composition in liquid form at room temperature, whereas it forms a hydrogel with beneficial properties once it encounters the higher temperatures associated with the eye.

Evidence of the effect of the hydrogel as such on symptoms and objective endpoints in dry eye disease is provided by the Examples that follow. In the context of a clinical trial designed to test the hydrogel as carrier for an added active ingredient, it was unexpectedly shown that the hydrogel as such is efficacious, even in the absence of an additional active ingredient. Furthermore, the effects observed with the hydrogel placebo in this study had a rapid onset of only 30 days. This is of significant benefit, in spite of the fact that the onset was not quite as rapid as the investigational product with an added active ingredient. The trial also showed that the hydrogel is safe for use.

Suitable hydrogel compositions are those described in US9592295, hereby incorporated by reference. In one embodiment, the water soluble block copolymer of the hydrogel is a tri-block copolymer having the general formula HO-(EO)ₐ-(PO)_{b}-(EO)ₐ-H, where (EO)ₐ is a polyethylene oxide block, (PO)_{b} is a polypropylene oxide block, a is in the range from 50 to 150, and b is in the range from 35 to 70. In a particular embodiment, a is about 101 and b is about 56 in such a tri-block copolymer. Such copolymers as those disclosed herein and in US9592295 are commonly known as "poloxamers", and the specific embodiment of a being about 101 and b being about 56 is known as Poloxamer 407.

The hydrogel composition also comprises at least one associative gelling agent. In one embodiment, the at least one associative gelling agent is selected from the group consisting of oxyalkylated fatty alcohol, esters of oxyalkylated fatty alcohol, oxyalkylated alkyl alcohol, esters of oxyalkylated alkyl alcohol, oxyalkylated alkylaryl alcohol, aliphatic hydroxy carboxylic acid, esters of aliphatic hydroxy carboxylic acid, aromatic hydroxy carboxylic acid, esters of aromatic hydroxy carboxylic acid, poly(hydroxy carboxylic acid), oxyalkylated sorbitan ester, oxyalkylated triglyceride, oxyalkylated glyceryl ester, esters of oxyalkylated sorbitol, polyol ester, sorbitan ester, and mixtures thereof. In a more specific embodiment, the at least one associative gelling agent is an ester of oxyalkylated fatty alcohol. Contemplated embodiments of such oxyalkylated fatty alcohols are for example selected from the group consisting of di-PPG-2 myreth-9 adipate, di-PPG-2 myreth-10 adipate and di-PPG-2 myreth-11 adipate. In a particular embodiment, said oxyalkylated fatty alcohol is di-PPG-2 myreth-10 adipate.

A suitable formulation of the hydrogel can be ascertained by a person of skill in the art of pharmaceutical formulations. Suitably, the pharmaceutical composition disclosed herein is formulated in such a way that local administration into the eye of a mammalian subject is facilitated. Thus, in one embodiment, the composition is administered in the form of an eye drop. In one such embodiment, it is provided in a single drop applicator, dispenser or bottle. In another such embodiment, it is provided in a multiple drop applicator, dispenser or bottle. In another embodiment, the composition is provided as a spray. In yet another embodiment, the composition is provided as an eyewash, or any other suitable means of administration into the ocular environment. In one embodiment, the composition is formulated and administered as an eye drop. As explained above, the reversely thermo-reversible properties of the composition ensure that it is in liquid form while refrigerated or at room temperature. This allows e.g. the administration of a drop of liquid into the eye. After administration in this way, the composition will gel when reaching the higher temperature in the eye.

In the clinical trial reported in the Examples to follow, the effect of the hydrogel *per* se was observed when it was administered twice daily. In one embodiment of the use and method aspects of the present disclosure, the composition is administered twice daily or more seldom, for example once daily or more seldom. In one specific embodiment, the composition is administered twice daily. In another specific embodiment, the composition is administered once daily.

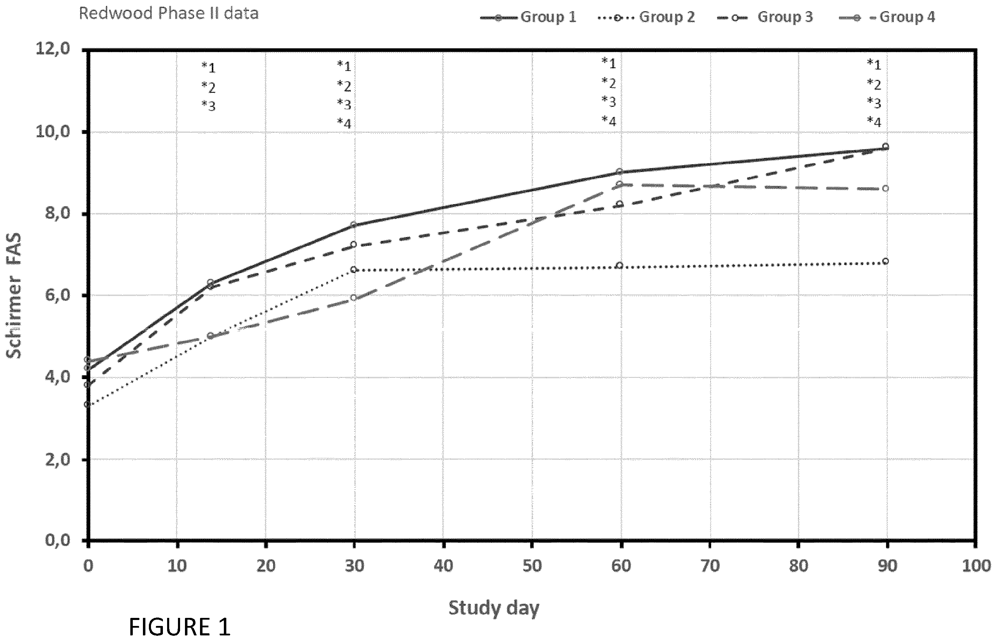

Throughout this disclosure, the use of the term dry eye disease is intended to broadly encompass any form of condition that exhibits a dry eye pathology, whether caused by reduced tear flow or not, and regardless of underlying cause. For an overview of various conditions and underlying causes that are all contemplated within the ambit of the disclosure, see Figure 1 of Craig *et al* (2017, *supra).* In the most general sense of the disclosure, the mammalian subject to be treated is not restricted to any particular species, sex, age group or other category. Thus, in one embodiment, the mammalian subject is male. In another embodiment, the mammalian subject is female. In one embodiment, the subject is a human being. In a specific such embodiment, the subject is a man. In another specific such embodiment, the subject is a woman. In a preferred embodiment, the dry eye disease is a consequence of hormone changes associated with menopause in a woman. Therefore, in one such embodiment, the mammalian subject to be treated is a woman undergoing menopause. In another such embodiment, the mammalian subject to be treated is a post-menopausal woman.

### Brief description of the drawings

Figure 1 is a diagram showing the evolution of Schirmer type II test scores ("Schirmer FAS") for study groups 1-4 over the course of the clinical trial described in Example 2. Group 1: solid line. Group 2: dotted line. Group 3: short dashes. Group 4: long dashes.
Figure 2 is a diagram showing the evolution of TFBUT ("tBUT") test scores for study groups 1-4 over the course of the clinical trial described in Example 2. Group 1: solid line. Group 2: dotted line. Group 3: short dashes. Group 4: long dashes.

### Examples

### Example 1

### Preparation of a reversely thermo-reversible hydrogel

A reversely thermo-reversible hydrogel was prepared using the below ingredients in the listed weight percentages. The thermogelling formulation was based on Poloxamer 407 and di-PPG-2 myreth-10 adipate as thermogelling agents.

| Component | % (w/w) |
|---|---|
| Poloxamer 407 | 12.9 |
| Di-PPG-2 myreth-10 adipate | 3.61 |
| Sodium phosphate monobasic dihydrate | 0.008 |
| Sodium phosphate dibasic dihydrate | 0.140 |
| HCl, 1M | Qs pH 7.4 |
| NaOH, 1M | Qs pH 7.4 |
| Water for injection | Qs 100 % |

Poloxamer 407 was dissolved in phosphate buffer at cold conditions, after which the temperature was raised to room temperature and di-PPG-2 myreth-10 adipate was added, creating a weight ratio of 78.1 : 21.9. Then, pH was adjusted to within the range from 7.2 to 7.6 by addition of 1 M HCl and/or 1 M NaOH. The resulting formulation was a mucoadhesive hydrogel, suitable for the treatment of dry eye disease by local administration in the eye. The hydrogel prepared above is denoted "IntelliGel" in the following Example and used as carrier or vehicle for an additional agent intended as active ingredient.

### Example 2

### Design of a clinical trial of active ingredient 17β-estradiol-3-phosphate in IntelliGel as carrier

A clinical trial was carried out with the purpose of testing the effect of 0.05 % and 0.1 % 17β-estradiol-3-phosphate ("E3P") on dry eye disease, when delivered via slow release from the IntelliGel hydrogel prepared in Example 1 as carrier. The tested drug product, comprising E3P in IntelliGel, was denoted RP101, and the clinical trial design is described on clinicaltrials.gov with the identifier NCT03821415.

In brief, the trial was a multicentre, randomized, double-masked, parallel-group, placebo-controlled Phase II study, in which the ophthalmic formulation RP101 was tested against IntelliGel only, as placebo.

### Study population

The study population consisted of 104 participants meeting a list of inclusion criteria that included:
- Sex and menopause: postmenopausal women; postmenopausal condition defined as final menstrual period at least 3 years before the screening
- Dry eye disease: patients with moderate to severe dry eye syndrome
- Schirmer's test with anesthesia ≤ 7 mm/5 min in the worse eye (study eye)
- Tear film breakup time: TFBUT ≤ 10 s in the worse eye (study eye)
- Visual acuity: corrected visual acuity ≥ 20/200 in each eye
- Symptoms: at least 2 of the typical dry eye disease symptoms since at least 3 months before the screening: foreign body sensation, burning/stinging, redness, tearing, pain, itching, blurred vision, photophobia, eyelid swelling, moisture and mucous discharge

Of the 104 enrolled participants, 77 completed the entire trial.

### Investigational product

The products tested in the trial were:

| | |
|---|---|
| TEST 1 (T1): | RP101 0.05 % |
| | 0.05 % w/w of E3P in IntelliGel |
| TEST 2 (T2): | RP101 0.1 % |
| | 0.1 % w/w of E3P in IntelliGel |
| PLACEBO (P): | IntelliGel according to Example 1, without E3P |

### Treatment groups

Subjects were randomly assigned in four equal parts to the following treatment groups, and received one of the treatments shown in Table 1 below for 90 consecutive days.

**Table 1**

| **Dose regimen** | **Morning** | **Evening** |
|---|---|---|
| **Group 1** | **T1** | **T1** |
| **Group 2** | **T2** | **P** |
| **Group 3** | **T2** | **T2** |
| **Group 4** | **P** | **P** |

### Objectives

The primary objective of the study was to establish the effective dose/dose regimen of RP101 in post-menopausal women with moderate to severe dry eye disease applying RP101 ophthalmic sterile solution or matching placebo (vehicle) once (q.d.) or twice a day (b.i.d.) for 3 months.

The secondary objectives of the study were to evaluate the safety and tolerability of the treatment; to evaluate as exploratory variables tear film osmolarity and corneal pachymetry in specific substudies; and to evaluate the pharmacokinetics (PK) of serum 17-β-estradiol after the first and last doses (PK substudy).

### Endpoints

The primary endpoint was to evaluate clinical efficacy during and at the end of the treatment with RP101 or matching placebo on the basis of measurements of Schirmer test type II (with anesthesia). Secondary endpoint parameters for evaluation of efficacy included:
- Visual analogue scale (VAS) for ocular tolerability (foreign body sensation, burning/stinging, itching, pain, sticky feeling, blurred vision, redness, tearing, eyelid swelling and photophobia)
- Symptom Assessment in Dry Eye (SANDE)
- Visual acuity (early treatment diabetic retinopathy study [ETDRS] chart)
- Slit lamp examination (SLE)
- Tear film osmolarity (only specific substudy)
- Tear film break up time (TFBUT)
- Fundus ophthalmoscopy
- Corneal fluorescein staining
- Corneal pachymetry (only specific substudy)
- Frequency of instillation (i.e. number of drops instilled daily) of artificial tears (rescue medication) during the treatment phase

### Schirmer test type II

This test was performed to measure basal aqueous tear secretion following the instillation of a preservative-free anesthetic eye drop. Both eyes may be tested at the same time. Schirmer's plus^{®} strips were used. The test was conducted in a dimly lit room. While the patient looked upwards, the lower lid was drawn gently downwards and temporally. The rounded bent end of a sterile strip was inserted into the lower conjunctival sac over the temporal one-third of the lower eyelid margin. The test was done without touching the Schirmer's test strip directly with the fingers, to avoid contamination by skin oils. Patients were instructed to close their eyes gently. After 5 min, the test strip was removed and the length of the tear absorption on the strip was measured (mm/5 min). The wetting distance after 5 min for each eye was recorded.

### Tear film break up time (TFBUT)

TFBUT was measured by determining the time to tear break-up, and performed after instillation of sodium fluorescein solution into the inferior conjunctival cul-de-sac of each eye. The patient was instructed to blink several times to thoroughly mix the fluorescein with the tear film. In order to achieve maximum fluorescence, the examiner waited approximately 30 s after instillation before evaluating TFBUT. With the aid of a slit lamp at 10X magnification using cobalt blue illumination, the examiner monitored the integrity of the tear film, noting the time it takes to form lacunae (clear spaces in the tear film) from the time that the eye is open after the last blink. The TFBUT was measured twice during the first minute after instillation of fluorescein. If the two readings differed by more than 2 s, a third reading was taken. The average of the 2 or 3 measurements was recorded as the TFBUT value.

### Example 3

### Unexpected efficacy shown in placebo group

The clinical trial described in Example 2 was conducted and the results evaluated. When analyzing the results, it was unexpectedly found that the subjects in group 4, treated with placebo only, exhibited outcomes on objective endpoints that indicate that the placebo as such, i.e. the IntelliGel hydrogel prepared as in Example 1, has a clinical efficacy in its own right, even without E3P added as active ingredient. Furthermore, the efficacy was characterized by a rapid onset, showing clear effects after only 30 days of the study.

### Schirmer test type II

The result of the Schirmer test for all four study groups is shown in Figure 1. As can be seen from this diagram, study group 4, which received IntelliGel placebo only, exhibited a comparable score to the groups that received the investigational product with different concentrations of E3P.

### Tear film break up time (TFBUT)

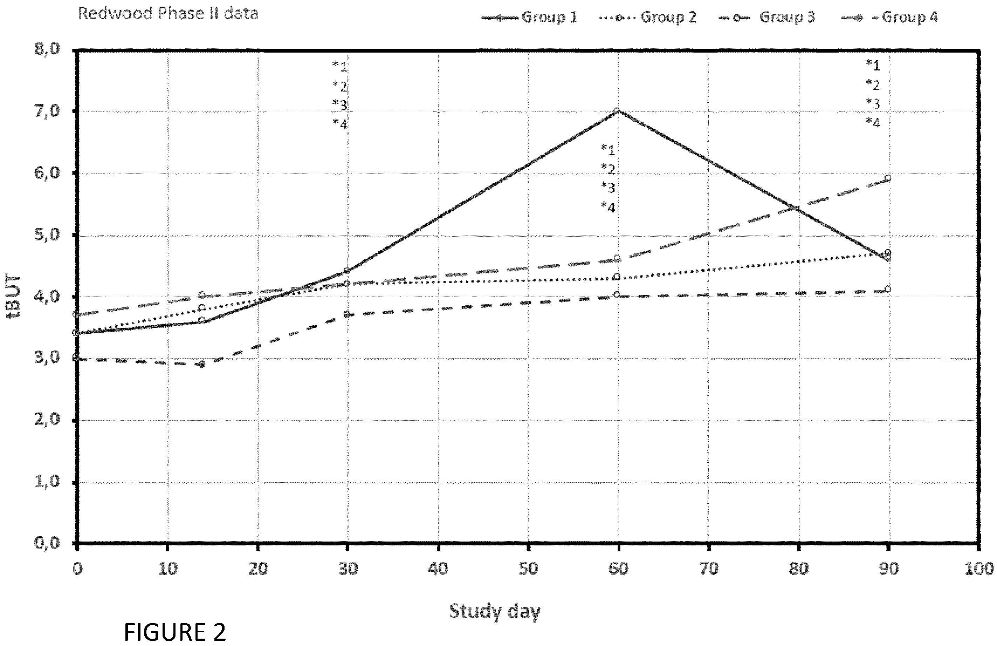

The result of the TFBUT test for all four study groups is shown in Figure 2. As can be seen from this diagram, study group 4, which received IntelliGel placebo only, exhibited a comparable score to the groups that received the investigational product with different concentrations of E3P. In fact, by day 90 of the study, the group receiving placebo exhibited a TFBUT score which was the highest of all study groups.

### Conclusion

The outcome of the clinical trial described in Example 2 shows that not only did the investigational product RP101 exhibit consistent and pronounced benefit in objective measures of dry eye disease, but such benefits could also be seen in the placebo group. Also, both the investigational product and the placebo were safe for use. In conclusion, it was shown that the observed benefits are achieved using only a hydrogel *per* se as "active ingredient" in its own right.

## Claims

1. Pharmaceutical composition, which comprises a reversely thermo-reversible hydrogel as active ingredient, said hydrogel comprising, by weight percent of the total composition:
- about 5-25 % of a water soluble block copolymer comprising at least two blocks of polyethylene oxide and at least one block of polypropylene oxide; and
- about 0.05-10 % of at least one associative gelling agent having a water solubility of less than 0.5 g / 100 ml at 20 °C and being capable of forming water soluble inter-molecular complexes with said water soluble block copolymers in water;
wherein said composition does not comprise any additional active ingredient,
for use in the treatment of dry eye disease by local administration in the eye of a mammalian subject in need thereof.

2. Pharmaceutical composition for use according to claim 1, wherein said water soluble block copolymer is a tri-block copolymer having the general formula HO-(EO)ₐ-(PO)_{b}-(EO)ₐ-H, where (EO)ₐ is a polyethylene oxide block, (PO)_{b} is a polypropylene oxide block, a is in the range from 50 to 150, and b is in the range from 35 to 70.

3. Pharmaceutical composition for use according to claim 2, where a is about 101, and b is about 56.

4. Pharmaceutical composition for use according to any preceding claim, wherein said at least one associative gelling agent is selected from the group consisting of oxyalkylated fatty alcohol, esters of oxyalkylated fatty alcohol, oxyalkylated alkyl alcohol, esters of oxyalkylated alkyl alcohol, oxyalkylated alkylaryl alcohol, aliphatic hydroxy carboxylic acid, esters of aliphatic hydroxy carboxylic acid, aromatic hydroxy carboxylic acid, esters of aromatic hydroxy carboxylic acid, poly(hydroxy carboxylic acid), oxyalkylated sorbitan ester, oxyalkylated triglyceride, oxyalkylated glyceryl ester, esters of oxyalkylated sorbitol, polyol ester, sorbitan ester, and mixtures thereof.

5. Pharmaceutical composition for use according to claim 4, wherein said at least one associative gelling agent is an ester of oxyalkylated fatty alcohol.

6. Pharmaceutical composition for use according to claim 5, wherein said oxyalkylated fatty alcohol is selected from the group consisting of di-PPG-2 myreth-9 adipate, di-PPG-2 myreth-10 adipate and di-PPG-2 myreth-11 adipate.

7. Pharmaceutical composition for use according to claim 6, wherein said oxyalkylated fatty alcohol is di-PPG-2 myreth-10 adipate.

8. Pharmaceutical composition for use according to any one of the preceding claims, in which said mammalian subject is a human patient.

9. Pharmaceutical composition for use according to claim 8, wherein said patient is a woman.

10. Pharmaceutical composition for use according to claim 9, wherein said woman is selected from the group consisting of a woman undergoing menopause and a post-menopausal woman.

## Patentansprüche

1. Pharmazeutische Zusammensetzung, die ein umgekehrt thermoreversibles Hydrogel als Wirkstoff umfasst, wobei das Hydrogel in Gewichtsprozent der gesamten Zusammensetzung Folgendes umfasst:
- etwa 5-25 % eines wasserlöslichen Blockcopolymers mit mindestens zwei Blöcken aus Polyethylenoxid und mindestens einem Block aus Polypropylenoxid und
- etwa 0,05-10 % mindestens eines assoziativen Geliermittels, das eine Wasserlöslichkeit von weniger als 0,5 g / 100 ml bei 20 °C aufweist und mit den wasserlöslichen Blockcopolymeren in Wasser wasserlösliche intermolekulare Komplexe bilden kann;
wobei die Zusammensetzung keinen zusätzlichen Wirkstoff umfasst,
zur Verwendung bei der Behandlung von trockenem Auge durch lokale Verabreichung im Auge eines Säugetierindividuums, bei dem diesbezüglicher Bedarf besteht.

2. Pharmazeutische Zusammensetzung zur Verwendung nach Anspruch 1, wobei es sich bei dem wasserlöslichen Blockcopolymer um ein Triblockcopolymer mit der allgemeinen Formel HO-(EO)ₐ-(PO)_{b}-(EO)ₐ-H handelt, wobei (EO)ₐ ein Polyethylenoxidblock ist, (PO)_{b} ein Polypropylenoxidblock ist, a im Bereich von 50 bis 150 liegt und b im Bereich von 35 bis 70 liegt.

3. Pharmazeutische Zusammensetzung zur Verwendung nach Anspruch 2, wobei a für etwa 101 steht und b für etwa 56 steht.

4. Pharmazeutische Zusammensetzung zur Verwendung nach einem der vorhergehenden Ansprüche, wobei das mindestens eine assoziative Gelierungsmittel aus der Gruppe bestehend aus oxyalkyliertem Fettalkohol, Estern von oxyalkyliertem Fettalkohol, oxyalkyliertem Alkylalkohol, Estern von oxyalkyliertem Alkylalkohol, oxyalkyliertem Alkylarylalkohol, aliphatischer Hydroxycarbonsäure, Estern von aliphatischer Hydroxycarbonsäure, aromatischer Hydroxycarbonsäure, Estern von aromatischer Hydroxycarbonsäure, Poly(hydroxycarbonsäure), oxyalkyliertem Sorbitanester, oxyalkyliertem Triglycerid, oxyalkyliertem Glycerylester, Ester von oxyalkyliertem Sorbitol, Polyolester, Sorbitanester und Mischungen davon ausgewählt ist.

5. Pharmazeutische Zusammensetzung zur Verwendung nach Anspruch 4, wobei es sich bei dem mindestens einen assoziativen Geliermittel um einen Ester von oxyalkyliertem Fettalkohol handelt.

6. Pharmazeutische Zusammensetzung zur Verwendung nach Anspruch 5, wobei der oxyalkylierte Fettalkohol aus der Gruppe bestehend aus Di-PPG-2-myreth-9-adipat, Di-PPG-2-myreth-10-adipat und Di-PPG-2-myreth-11-adipat ausgewählt ist.

7. Pharmazeutische Zusammensetzung zur Verwendung nach Anspruch 6, wobei es sich bei dem oxyalkylierten Fettalkohol um Di-PPG-2-myreth-10 adipat handelt.

8. Pharmazeutische Zusammensetzung zur Verwendung nach einem der vorhergehenden Ansprüche, wobei es sich bei dem Säugetierindividuum um einen menschlichen Patienten handelt.

9. Pharmazeutische Zusammensetzung zur Verwendung nach Anspruch 8, wobei es sich bei dem Patienten um eine Frau handelt.

10. Pharmazeutische Zusammensetzung zur Verwendung nach Anspruch 9, wobei die Frau aus der Gruppe bestehend aus einer Frau in der Menopause und einer postmenopausalen Frau ausgewählt ist.

## Revendications

1. Composition pharmaceutique, qui comprend un hydrogel thermo-réversible inversement comme ingrédient actif, ledit hydrogel comprenant, en pourcentage en poids de la composition totale :
- environ 5-25 % d'un copolymère séquencé soluble dans l'eau comprenant au moins deux blocs de poly(oxyde d'éthylène) et au moins un bloc de poly(oxyde de propylène) ; et
- environ 0,05-10 % d'au moins un agent gélifiant associatif ayant une solubilité dans l'eau inférieure à 0,5 g/100 ml à 20 °C et capable de former des complexes intermoléculaires hydrosolubles avec lesdits copolymères séquencés hydrosolubles dans l'eau ;
dans laquelle ladite composition ne comprend aucun ingrédient actif supplémentaire,
pour une utilisation dans le traitement de la sécheresse oculaire par administration locale dans l'œil d'un sujet mammifère qui en a besoin.

2. Composition pharmaceutique pour une utilisation selon la revendication 1, dans laquelle ledit copolymère séquencé soluble dans l'eau est un copolymère triséquencé ayant la formule générale HO-(EO)ₐ-(PO)_{b}-(EO)ₐ-H, où (EO)ₐ est un bloc de poly(oxyde d'éthylène), (PO)_{b} est un bloc de poly(oxyde de propylène), a est dans la plage de 50 à 150, et b est dans la plage de 35 à 70.

3. Composition pharmaceutique pour une utilisation selon la revendication 2, dans laquelle a est d'environ 101 et b est d'environ 56.

4. Composition pharmaceutique pour une utilisation selon l'une quelconque des revendications précédentes, dans laquelle ledit au moins un agent gélifiant associatif est choisi dans le groupe constitué par l'alcool gras oxyalkylé, les esters d'alcool gras oxyalkylé, l'alcool alkylique oxyalkylé, les esters d'alcool alkylique oxyalkylé, l'alcool alkylarylique oxyalkylé, l'acide hydroxycarboxylique aliphatique, les esters d'acide hydroxycarboxylique aliphatique, l'acide hydroxycarboxylique aromatique, les esters d'acide hydroxycarboxylique aromatique, le poly(acide hydroxycarboxylique), l'ester de sorbitane oxyalkylé, le triglycéride oxyalkylé, l'ester de glycéryle oxyalkylé, les esters de sorbitol oxyalkylé, l'ester de polyol, l'ester de sorbitane et leurs mélanges.

5. Composition pharmaceutique pour une utilisation selon la revendication 4, dans laquelle ledit au moins un agent gélifiant associatif est un ester d'alcool gras oxyalkylé.

6. Composition pharmaceutique pour une utilisation selon la revendication 5, dans laquelle ledit alcool gras oxyalkylé est choisi dans le groupe constitué par l'adipate de di-PPG-2 myreth-9, l'adipate de di-PPG-2 myreth-10 et l'adipate de di-PPG-2 myreth-11.

7. Composition pharmaceutique pour une utilisation selon la revendication 6, dans laquelle ledit alcool gras oxyalkylé est l'adipate de di-PPG-2 myreth-10.

8. Composition pharmaceutique pour une utilisation selon l'une quelconque des revendications précédentes, dans laquelle ledit sujet mammifère est un patient humain.

9. Composition pharmaceutique pour une utilisation selon la revendication 8, dans laquelle ledit patient est une femme.

10. Composition pharmaceutique pour une utilisation selon la revendication 9, dans laquelle ladite femme est choisie dans le groupe constitué par une femme ménopausée et une femme post-ménopausée.
